# EUROPEAN PATENT SPECIFICATION

(11) **EP 0 383 432 B1**
(45) Date of publication and mention of the grant of the patent: **24.03.1993**
(21) Application number: 90300476.0
(22) Date of filing: 17.01.1990
(51) Int. Cl.: A61K 31/22

(54) **Coenzyme Q10 with HMG-COA reductase inhibitors**
Koenzym Q10 mit HMG-COA-Reduktase-Inhibitoren
Coenzyme Q10 avec inhibiteurs de HMG-COA réductase

(30) Priority: 18.01.1989 US 298535; 02.02.1989 US 305140
(43) Date of publication of application: 22.08.1990
(73) Proprietor: Merck & Co., Inc., Rahway New Jersey 07065-0900 (US)
(72) Inventor: Brown, Michael S., Dallas, Texas 75209 (US); Tobert, Jonathan A., Maplewood, New Jersey 07040 (US)
(74) Representative: Cole, William Gwyn

(56) References cited:
- BIOLOGICAL ABSTRACTS, vol. 87, no. 2, abstract no. 14184, Philadelphia, US; J.W. DOYLE et al.: "Requirement for mevalonate in cycling cells: Quantitative and temporal aspects"

## Description

### BACKGROUND OF THE INVENTION

Coenzyme Q₁₀ (2,3-dimethoxy-5-methyl-6-decaprenyl-1,4-benzoquinone) is a redox component in the respiratory chain and is found in all cells having mitochondria. It is thus an essential co-factor in the generation of metabolic energy and is particularly important in muscle function. For example, Folkers et al., Proc. Natl. Acad. Sci., 82: 901 (1985) have measured the levels of Coenzyme Q₁₀ (CoQ₁₀) in endomyocardial biopsy samples taken from patients with varying stages of cardiomyopathy. Folkers et al. states that these data show decreasing tissue levels of CoQ₁₀ with increasing severity of the symptoms of cardiac disease. Folkers et al., Proc. Natl. Acad. Sci., 82: 4513 (1985) in a double-blind study have reported improved cardiac output for some patients upon receiving an oral administration of CoQ₁₀.

HMG-CoA reductase inhibitors represent a new class of cholesterol-lowering drugs. Relatively low doses of these drugs effectively reduce plasma cholesterol levels. These drugs are believed to function by inhibiting the chemical transformation HMG-CoA to mevalonate, which is an early and rate-limiting step in the biosynthesis of cholesterol. A branch of the mevalonate cholesterol biosynthetic pathway in mammalian cells leads to the formation of CoQ₁₀.[reviewed by Brown and Goldstein J. Lipid Res., 21, 505(1980)]. Furthermore high levels of lovastatin can reduce CoQ₁₀ in the liver (MK-803 NDA report) and compactin reduces LDL-bound CoQ₁₀ at doses employed in humans[H. Mabuchi et al, N. E. J. Med., 478(August 1981].

The Physician's Desk Reference, 42d Ed., 1366 (1988) states that skeletal muscle myalgia has been associated with lovastatin therapy. Tobert, N.E.J. Med., 48 (Jan. 7, 1988) states that in a very small number of patients (0.5 percent) myopathy appeared to be associated with lovastatin therapy. Concomitant therapy with immunosuppressant drugs, including cyclosporine, with gemfibrozil or niacin or a combination, appears to increase the risk of myopathy. (J.A. Tobert, Am. J. Cardiol. 1988, 62: 28J-34J). The myopathy is reversible upon discontinuance of lovastatin therapy. About 1.9% of the patients treated with lovastatin in clinical trials have had asymptomatic but marked and persistent transaminase increases, particular serum glutamic pyruvic transaminase. (J. A. Tobert, Am. J. Cardiol., 1988 62: 28J-34J). The elevated transaminase level is reversible upon discontinuance of the therapy.

Although cholesterol-lowering therapy through the use of HMG-CoA reductase inhibitors is generally free of side reactions, it would be of considerable benefit to counteract the myopathy and increased transaminase levels observed in a small percent of patients. Since CoQ₁₀ is of benefit in congestive heart failure patients the combination with HMG-CoA reductase inhibitors should be of value in such patients who also have the added risk of high cholesterol levels.

### DETAILED DESCRIPTION OF THE INVENTION

The present invention concerns the use of an HMG-CoA reductase inhibitor and Coenzyme Q₁₀ for the preparation of a medicament adapted for adjunct administration of an HMG-CoA reductase inhibitor and an effective amount of Coenzyme Q₁₀ for counteracting HMG-CoA reductase inhibitor associated skeletal muscle myopathy in a patient receiving HMG-CoA reductase therapy. The invention also relates to the use of an HMG-CoA reductase inhibitor and Coenzyme Q₁₀ for the preparation of a medicament, adapted for adjunct administration of a HMG-CoA reductase inhibitor and an effective amount of Coenzyme Q₁₀ for counteracting HMG-CoA reductase inhibitor-associated liver damage in a patient receiving HMG-CoA reductase therapy.

The HMG-CoA reductase inhibitor employed may be lovastatin, simvastatin, pravastatin, XU-62-320 (Sodium-3,5-dihydroxy-7-[3-(4-fluorophenyl)-1(methylethyl)-1H-lndole-2yl]-hept-6-enoate) or any other member of the class of compounds that inhibit HMG-CoA reductase. The preparation of lovastatin (U.S. Patent 4,231,938), simvastatin (U.S. Patent 4,444,784) and pravastatin (U.S. Patent 4,346,227) have been described in the patent literature. The preparation of XU-62-320 is described in WIPO Patent WO84/02131, published June 7, 1984.

Coenzyme Q₁₀ is manufactured by the Kanegafuchi Chemical Industry Co., Ltd. and is widely available.

In its application to the counteraction of myopathy and of liver damage, particularly elevated transaminase levels, the present invention is accordingly to be understood as providing for the avoidance of myopathy and liver damage or elevated transaminase levels where this may otherwise occur as well as the amelioration of myopathy, liver damage and elevated transaminase levels. The term counteracting is accordingly to be understood as connecting both a precautionary or prophylactic as well as curative or treatmental function.

The compounds of the instant invention may be administered orally or parenterally in the form of a capsule, a tablet, an injectable preparation or the like. The general amounts of HMG-CoA reductase inhibitor will be of the same or similar order to that employed in HMG-CoA reductase therapy. In general, satisfactory results are obtained by administration of .10 to 80 mg/day of the HMG-CoA reductase inhibitor in a single or divided dose. Doses of CoQ₁₀ may vary from 25 mg to 1 g day in a single or divided dose. Tablets or capsules may also be administered which contain both compounds in the dosage ranges indicated.

### EXAMPLE 1

As a specific embodiment of a composition of this invention, 20 mg of lovastatin and 35 mg of Coenzyme Q₁₀ are formulated with sufficient finely-divided lactose to provide a total amount of 580 to 590 mg to fill a size 0 hard-gelatin capsule. Optionally added are a excipient such as finely divided cellulose, a disintegrant such as Explotat® and a lubricant such as magnesium stearate.

## Claims

1. A pharmaceutical antihypercholesterolemic composition capable of counteracting skeletal muscle myopathy comprising a pharmaceutical carrier and an effective amount of a HMG-CoA reductase inhibitor and an effective amount of Coenzyme Q₁₀.

2. A composition of Claim 1 in which the HMG-CoA reductase inhibitor is selected from:
lovastatin, simvastatin, pravastatin or XU-62-320.

3. The use of an HMG-CoA reductase inhibitor and Coenzyme Q₁₀ for the preparation of a medicament adapted for adjunct administration of an HMG-CoA reductase inhibitor and an effective amount of Coenzyme Q₁₀ for counteracting HMG-CoA reductase associated skeletal muscle myopathy in a patient receiving HMG-CoA reductase therapy.

4. The use as claimed in Claim 3 in which the HMG-CoA reductase inhibitor is selected form the group consisting of:
lovastatin, simvastatin, pravastatin, or Xu-62-30.

5. The use of an HMG-CoA reductase inhibitor and coenzyme Q₁₀ for the preparation of a medicament adapted for adjunct administration of an HMG-CoA reductase inhibitor and an effective amount of coenzyme Q₁₀ for counteracting HMG-CoA reductase-associated elevated transaminase levels in a patient receiving HMG-CoA reductase therapy.

## Patentansprüche

1. Pharmazeutisches, antihypercholesterinämisches Präparat, das in der Lage ist,der Skelettmuskel-Myopatie entgegenzuwirken, umfassend einen pharmazeutischen Träger und eine wirksame Menge HMG-CoA Reduktase-Inhibitor sowie eine wirksame Menge Coenzym Q₁₀.

2. Präparat nach Anspruch 1, bei dem der HMG-CoA Reduktase-Inhibitor aus Lovastatin, Simvastatin, Pravastatin oder XU-62-320 ausgewählt ist.

3. Verwendung eines HMG-CoA Reduktase-Inhibitors und von Coenzym Q₁₀ zur Herstellung eines Medikaments, das für die zusätzliche Verabreichung eines HMG-CoA Reduktase-Inhibitors und einer wirksamen Menge Coenzym Q₁₀ geeignet ist, um der Skelettmuskel-Myopatie, die mit der HMG-CoA Reduktase einhergeht, mittels einer Therapie entgegenzuwirken, bei der dem Patienten HMG-CoA Reduktase verabreicht wird.

4. Verwendung nach Anspruch 3, bei der der HMG-CoA Reduktase-Inhibitor aus der Gruppe bestehend aus Lovastatin, Simvastatin, Pravastatin oder XU-62-30 ausgewählt wird.

5. Verwendung eines HMG-CoA Reduktase-Inhibitors und von Coenzym Q₁₀ zur Herstellung eines Medikaments, das für die zusätzliche Verabreichung eines HMG-CoA Reduktase-Inhibitors und einer wirksamen Menge Coenzym Q₁₀ geeignet ist, um den mit HMG-CoA Reduktase einhergehenden erhöhten Transaminasespiegeln mittels einer Therapie entgegenzuwirken, bei der dem Patiencen HMG-CoA Reduktase verabreicht wird.

## Revendications

1. Composition pharmaceutique antihypercholestérolémique capable de contrecarrer la myopathie des muscles squelettiques, qui comprend un véhicule pharmaceutique et une quantité efficace d'un inhibiteur de l' HMG-CoA réductase et une quantité efficace de Coenzyme Q₁₀.

2. Composition selon la revendication 1, dans laquelle l'inhibiteur de l'HMG-CoA réductase est choisi parmi : la lovastatine, la simvastatine, la pravastatine ou XU-62-320.

3. Utilisation d'un inhibiteur de l'HMG-CoA réductase et de la Coenzyme Q₁₀ pour la préparation d'un médicament adapté à l'administration complémentaire d'un inhibiteur de l'HMG-CoA réductase et d'une quantité efficace de Coenzyme Q₁₀ pour contrecarrer la myopathie des muscles squelettiques associée à l'HMG-CoA réductase chez un patient recevant une thérapie de l'HMG-CoA réductase.

4. Utilisation selon la revendication 3, dans laquelle l'inhibiteur de l'HMG-CoA réductase est choisi dans le groupe comprenant la lovastatine, la simvastatine, la pravastatine ou XU-62-30.

5. Utilisation d'un inhibiteur de l'HMG-CoA réductase et de la Coenzyme Q₁₀ pour la préparation d'un médicament adapté à l'administration complémentaire d'un inhibiteur de l'HMG-CoA réductase et d'une quantité efficace de Coenzyme Q₁₀ pour contrecarrer les niveaux élevés de transaminases associés à l'HMG-CoA réductase, chez un patient recevant une thérapie de l'HMG-CoA réductase.
